# EUROPEAN PATENT SPECIFICATION

(11) **EP 1 210 942 B1**
(45) Date of publication and mention of the grant of the patent: **21.03.2007**
(21) Application number: 02075905.6
(22) Date of filing: 06.05.1997
(51) Int. Cl.: A61K 9/16, A61K 9/50, A61K 31/517

(54) **Microparticles**
Micropartikel
Microparticules

(30) Priority: 07.05.1996 US 643919
(43) Date of publication of application: 05.06.2002
(62) Divisional of application: 97923063.8
(73) Proprietor: Alkermes, Inc., Cambridge MA 02139 (US); JANSSEN PHARMACEUTICA N.V., 2340 Beerse (BE)
(72) Inventor: Rickey, Michael E., Loveland, Ohio 45140 (US); Ramstack, J. Michael, Lebanon, OH 45036 (US); Lewis, Danny H., Hartselle, AL 35640 (US); Mesens, Jean Louis, 2275 Wechelderzande (BE)
(74) Representative: Van Borm, Werner August H.M.

(56) References cited:
- EP-A- 0 585 151
- EP-A- 0 669 128
- WO-A-95/11009
- WO-A-95/13799
- WO-A-95/13814
- US-A- 4 389 330
- US-A- 5 478 564
- LEROUW J-C ET AL: "NEW APPROACH FOR THE PREPARATION OF NANOPARTICLES BY AN EMULSIFICATION-DIFFUSION METHOD" EUROPEAN JOURNAL OF PHARMACEUTICS AND BIOPHARMACEUTICS, ELSEVIER SCIENCE PUBLISHERS B.V., AMSTERDAM, NL, vol. 41, no. 1, 1995, pages 14-18, XP000482886 ISSN: 0939-6411
- BUDAVARI S.: "MERCK INDEX. 11th ed." 1989 , MERCK & CO. , RAHWAY, N.J. U.S.A. XP002275712 166630 * page 1137 *

## Description

This invention relates to microparticles having an improved storage stability. More particularly, the present invention relates to pharmaceutical compositions comprising controlled-release microparticles having improved shelf-life, said microparticles comprising active agents encapsulated within a polymeric matrix.

Compounds can be encapsulated in the form of microparticles (e.g. particles having a mean size in the nanometer to millimeter range, particularly in the range 1 to 500 µm, especially 25 to 180 µm) by a variety of known methods. It is particularly advantageous to encapsulate a biologically active or pharmaceutically active agent within a biocompatible, biodegradable, wall-forming material (e.g. a polymer) to provide sustained or delayed release of drugs or other active agents. In these methods, the material to be encapsulated (a drug or other active agent) is generally dissolved, dispersed, or emulsified, using known mixing techniques, in a solvent containing the wall-forming material. Solvent is then removed from the microparticles and thereafter the microparticle product is obtained.

Very often the solvents used in the known microencapsulation processes are halogenated hydrocarbons, particularly chloroform or methylene chloride, which act as solvents for both the active agent and the encapsulating polymer. The presence of small, but detectable, halogenated hydrocarbon residuals in the final product, however, is undesirable, because of their general toxicity and possible carcinogenic activity.

In WO-95/13799, a process was disclosed for preparing biodegradable, biocompatible microparticles comprising a biodegradable, biocompatible polymeric binder and a biologically active agent, wherein a blend of at least two substantially non-toxic solvents, free of halogenated hydrocarbons, was used to dissolve both the agent and the polymer. This solvent blend was dispersed in an aqueous solution to form an emulsion that was then added to an aqueous extraction medium which preferably contained at least one of the solvents of the blend, whereby the rate of extraction of each solvent was controlled, whereupon biodegradable, biocompatible microparticles containing the biologically active agent were formed.

WO95/13799 describes microparticles comprising a biodegradable matrix and a biologically active agent. Residual solvent levels are however, very high in these particles.

EP-A-585151 concerns the formulation of LHRH in microparticles but employs halogenated solvents to achieve this.

In WO95/11009, the inventors seek to prevent microparticles bursting during drying thereof. Residual solvent levels in these particles are again very high.

In the European Journal of Pharmaceutics and Biopharmaceutics, 41 (1995), Jan, No. 1, Leroux et al describe nanoparticles which can be loaded with various active agents.

Risperidone encapsulated in microparticles prepared using a benzyl alcohol and ethyl acetate solvent system is also described in WO95/13814.

These microparticulate products however have been found to degrade during storage. A need therefore exists for a means by which the degradation rate can be reduced, thereby increasing the shelf-life of the product and enhancing its commercial feasibility.

It has now surprisingly been found that the rate of product degradation can be reduced by reducing the level of residual processing solvent. It is thought that one degradation process that was occurring, resulted, at least in part, from hydrolysis of the polymeric matrix, and that the rate of hydrolysis was directly influenced by the level of residual processing solvent (e.g. benzyl alcohol) in the product. By reducing the level of residual solvent in the microparticles, the rate of degradation is reduced, thereby increasing shelf-life.

Thus according to one aspect the invention provides microparticles of a biodegradable biocompatible polymeric matrix containing an active agent and an organic solvent free from halogenated hydrocarbons, said organic solvent being present in said microparticles at 2% or less of the total weight of said microparticles wherein the active agent is selected from the group of risperidone, 9-hydroxy-risperidone, and pharmaceutically acceptable salts thereof.

In an alternative aspect, the invention provides microparticles as hereinbefore defined, said microparticles being prepared by a process comprising either:
(a) contacting said microparticles with water and maintaining said water at a temperature in the range of from 25°C to 40°C during at least part of the time that it is in contact with said microparticles,
   or:
(b) contacting said microparticles with an aqueous solvent system comprising water and a water-miscible solvent for said organic solvent.

In a still further aspect, the invention provides a pharmaceutical composition comprising microparticles according to the invention, together with at least one pharmaceutically acceptable carrier or excipient.

Viewed from a further aspect the invention provides the use of microparticles of the invention for the manufacture of a medicament for use in a method of diagnosis or therapy.

In the invention, the initial content of organic solvent in the particles will generally be above 3.5%, more generally above 4.0% of the total weight of the particles. Advantageously, the invention will reduce this content to less than 2%, preferably to less than 1.5% and most preferably less than 1%. The organic solvent in question preferably contains a hydrophobic group containing at least 5 carbons, e.g. an aryl group such as a naphthyl or more especially a phenyl group.

The organic solvent in the particles will generally be present as a result of a particle formation process where the particles have been produced from a solution of the matrix forming polymer material in the organic solvent or in a solvent mixture or blend containing the organic solvent. The organic solvent will be a non-halogenated solvent and particularly preferably will be an at least partially water-miscible solvent such as an alcohol (e.g. benzyl alcohol), a linear or cyclic ether, a ketone or an ester (e.g. ethyl acetate). Where the organic solvent is one solvent in such a solvent mixture or blend any other solvent in the mixture or blend will preferably be a non-halogenated solvent and particularly preferably will be an at least partially water-miscible solvent such as an alcohol (e.g. a C₁₋₄ alkanol such as ethanol), a linear or cyclic ether, a ketone or an ester.

Where used, the water-miscible solvent in the aqueous solvent system (ie. the washing fluid) will likewise preferably be a non-halogenated solvent and particularly preferably will be an at least partially water-miscible solvent such as an alcohol (e.g. a C₁₋₄ alkanol such as ethanol), a linear or cyclic ether, a ketone or an ester.

The contacting with the aqueous solvent system may be effected in one or more stages, e.g. a single contact or a series of washes, optionally with differently constituted aqueous solvent systems. Preferably, the total contact time is for a period of ten minutes to several hours, e.g. 1 to 48 hours.

The matrix forming polymer material should of course have sufficiently limited solubility in the aqueous solvent system used that the particles do not dissolve completely in the solvent system during the contact period.

Particularly preferably the particles of the invention are prepared by producing a two phase liquid system in which a first discontinuous liquid phase is present in a second continuous liquid phase. The first liquid phase comprises the matrix forming polymer dissolved in a first solvent system in which the active agent is dissolved or dispersed. The first solvent system comprises the organic solvent optionally and preferably together with one or more co-solvents, the various solvents preferably being alcohols, ethers, esters or ketones and preferably not including any halogenated solvents. Preferably one of the solvents in the first solvent system has a hydrophilic group, e.g. an aryl group such as a phenyl group; particularly preferably it is benzyl alcohol. Preferably a second solvent with a higher water solubility, e.g. ethyl acetate, is present in the first solvent system. The second liquid phase preferably comprises one or more solvents, such as water, and is preferably such that the polymer is less soluble therein than in the first solvent system but such that the solvents of the first solvent system are at least partially soluble therein so allowing particles to form by diffusion of solvent from the first liquid phase into the second liquid phase. The second liquid phase advantageously may contain a hydrocolloid or a surfactant.

The process may be carried out using pre-formed particles or, more preferably, may additionally comprise the production of the particles, conveniently using a liquid phase containing as a solvent or co-solvent the organic solvent referred to above as well as the matrix forming polymer and the active agent. Particle formation may then be effected for example by spray drying or, more preferably, by forming an emulsion using a second liquid phase, e.g. an aqueous phase, with the first liquid phase being discontinuous and the second being continuous, i.e. as described above.

The present invention provides an improved pharmaceutical composition in microparticle form designed for the controlled release of an effective amount of a drug over an extended period of time, whereby the composition exhibits increased shelf-life. The useful shelf-life can be increased to about two or more years for microparticles of the present invention.

In one preferred embodiment, the process for preparing microparticles of the present invention comprises:
A) preparing a first phase comprising:
   (1) a biodegradable, biocompatible polymeric encapsulating binder, and
   (2) an active agent having limited water solubility, dissolved or dispersed in a first solvent;
B) preparing an aqueous second phase;
C) combining said first phase and said second phase under the influence of mixing means to form an emulsion in which said first phase is discontinuous and said second phase is continuous;
D) separating said discontinuous first phase from said continuous second phase; and
E) washing said discontinuous first phase with
   (1) water at a temperature in the range of from about 25°C to about 40°C, or
   (2) an aqueous solution comprising water and a second solvent for residual first solvent in said first phase,
thereby reducing the level of residual first solvent to less than about 2% by weight of said microparticles.

In a preferred aspect of the above-described process, a quench step is additionally performed between steps C) and D).

The aqueous second phase can be an aqueous solution of a hydrophilic colloid or a surfactant. The aqueous second phase can be water.

In another preferred embodiment, the process for preparing microparticles of the present invention comprises: preparing a first discontinuous phase (also referred to herein as an "oil phase" or an "organic phase") containing from about 5 weight percent to about 50 weight percent solids of which from about 5 to about 95 weight percent is a solution of biodegradable, biocompatible polymeric encapsulating binder and incorporating from about 5 to about 95 weight percent, as based on polymeric binder, of an active agent in a solvent blend, the blend comprising first and second mutually miscible co-solvents, each having a solubility in water of from about 0.1 to about 25 weight percent at 20°C; forming an emulsion containing 1 part by weight of the first phase in from 1 to 10 parts by weight of an emulsion process medium whereby to form microdroplets of the discontinuous first phase composition in a continuous or "aqueous" second phase process medium; adding the combined first and second phases to an aqueous extraction quench liquid at a level of from about 0.1 to about 20 liters of aqueous quench liquid per gram of polymer and active agent, the quench liquid containing the more water soluble co-solvent of the blend at a level of from about 20% to about 70% of the saturation level of the more water soluble co-solvent in the quench liquid at the temperature being used; recovering microparticles from the quench liquid; and washing the discontinuous first phase with water at an elevated temperature (i.e. above room temperature) or with an aqueous solution comprising water and a solvent for residual solvent in the first phase, thereby reducing the level of residual solvent in the microparticles. The level of residual solvent in the microparticles is preferably reduced to about 2% by weight of the microparticles.

In another preferred embodiment, the process for preparing microparticles of the present invention comprises:
A) preparing a first phase comprising
   1) a biodegradable, biocompatible polymeric encapsulating binder selected from poly(glycolic acid), poly-d,l-lactic acid, poly-l-lactic acid, and copolymers of the foregoing, and
   2) an active agent selected from risperidone, 9-hydroxy risperidone and pharmaceutically acceptable salts thereof, dissolved or dispersed in a blend comprising ethyl acetate and benzyl alcohol, said blend being free from halogenated hydrocarbons;
B) preparing a second phase comprising polyvinyl alcohol dissolved in water;
C) combining said first phase and said second phase in a static mixer to form an emulsion in which said first phase is discontinuous and said second phase is continuous;
D) immersing said first and said second phases in a quench liquid;
E) isolating said discontinuous first phase in the form of microparticles; and
F) washing said discontinuous first phase with an aqueous solution comprising water and ethanol, thereby reducing the level of benzyl alcohol to less than about 2% by weight of said microparticles.

In another preferred embodiment, the process for preparing microparticles of the invention comprises:
A) preparing a first phase, said first phase comprising an active agent (e.g. a biologically active agent), a biodegradable, biocompatible polymer, and a first solvent;
B) preparing a second phase in which said first phase is substantially immiscible;
C) flowing said first phase through a static mixer at a first flow rate;
D) flowing said second phase through said static mixer at a second flow rate so that said first phase and said second phase flow simultaneously through said static mixer thereby forming microparticles containing said active agent;
E) isolating said microparticles; and
F) washing said microparticles with water at an elevated temperature or with an aqueous solution comprising water and a second solvent for residual first solvent in said microparticles, thereby reducing the level of residual first solvent to less than about 2% by weight of said microparticles.

In further embodiments of the invention, the first phase may be prepared by dissolving a biologically active agent in a solution of the polymer dissolved in a solvent free from halogenated hydrocarbons and preparing a dispersion comprising the active agent in the polymer solution or preparing an emulsion comprising the active agent in the polymer solution.

In another aspect, the present invention relates to a pharmaceutical composition comprising biodegradable and biocompatible microparticles in a pharmaceutically acceptable carrier. The microparticles comprise a polymeric encapsulating binder having dispersed or dissolved therein an active agent, and less than about 2% by weight residual solvent, wherein the residual solvent is residual derived from a solvent employed in the preparation of the microparticles.

In another aspect, the present invention relates to a pharmaceutical composition comprising biodegradable and biocompatible microparticles, ranging in size from about 25 to about 180 microns, in a pharmaceutically acceptable carrier. The microparticles comprise a copolymer of poly(glycolic acid) and poly(d,l-lactic acid) wherein the molar ratio of lactide to glycolide is in the range of from about 85:15 to about 50:50 and having dispersed or dissolved therein from about 35 to about 40% of an active agent comprising risperidone or 9-hydroxy-risperidone, and from about 0.5 to about 1.5% by weight of benzyl alcohol.

An advantage of the process for preparing microparticles of the present invention is that it can be used to produce, *inter alia,* a biodegradable, biocompatible system that can be injected into a patient. The process makes it possible to mix microparticles containing different drugs, to produce microparticles free from halogenated hydrocarbon residues, and to program the release to give faster or slower rates of drug release as needed (ie. it is possible to achieve a multiphasic release pattern). Moreover, using the process one achieves improved shelf-life stability resulting from lowered residual solvent in the finished product.

An advantage of the products of the present invention is that durations of action ranging from 7 to more than 200 days, e.g. from 14 to 100 days can be obtained, depending upon the type of microparticle selected. In preferred embodiments, the microparticles may be designed to afford treatment to patients during duration of action periods of 14 to 60 days, 20 to 60 days, 30 to 60 days and 60 to 100 days. A 90 day duration of action period is considered to be particularly advantageous. The duration of action can be controlled by manipulation of the polymer composition, polymer:drug ratio, microparticle size, and concentration of residual solvent remaining in the microparticle after treatment.

Another important advantage of the microparticles of the present invention is that practically all of the active agent is delivered to the patient because the polymer used in the method of the present invention is biodegradable, thereby permitting all of the entrapped active agent to be released into the patient.

Still another important advantage of the microparticles of the present invention is that residual solvent(s) in the finished microparticle can be reduced by approximately an order of magnitude whereby the useful shelf-life of the product can be increased from about six months for product made without the washing (ie. contacting) step of the present invention to about two or more years for particles made with the washing step.

A further advantage of the process for preparing microparticles of the present invention is that it may prove beneficial in controlling the release characteristics of active agent *in vivo* or reducing an undesirable or possibly harmful solvent.

To ensure clarity of the description that follows, the following definitions are provided. By "microparticles" or "microspheres" is meant solid particles that contain an active agent dispersed or dissolved within a biodegradable, biocompatible polymer that serves as the matrix of the particle. By "limited water solubility" is meant having a solubility in water in the range of from about 0.1 to about 25 wt. % at 20°C. By "halogenated hydrocarbons" is meant halogenated organic solvents, e.g. C₁-C₄ halogenated alkanes, for example methylene chloride, chloroform, methyl chloride, carbon tetrachloride, ethylene dichloride, ethylene chloride, 2,2,2-trichloroethane, and the like. By "biodegradable" is meant a material that should degrade by bodily processes to products readily disposable by the body and should not accumulate harmfully in the body. The products of the biodegradation should also be biocompatible with the body. By "biocompatible" it is meant that the material concerned is not toxic to the human body, is pharmaceutically acceptable, is not carcinogenic, and does not significantly induce inflammation in body tissues. By "weight %" or "% by weight" is meant parts by weight per total weight of microparticle. For example, 10 wt. % agent would mean 10 parts agent by weight and 90 parts polymer by weight. Unless otherwise specified, percentages referred to herein are by weight unless it is clear from the context that this is not the case.

In the process for preparing microparticles of the present invention, a solvent free from halogenated hydrocarbons may be used to produce biodegradable, biocompatible microparticles comprising at least one biologically active agent. A particularly preferred solvent is a solvent blend comprising at least two solvents. A first solvent component of the solvent blend is preferably a poor solvent for the active agent but a good solvent for the biodegradable, biocompatible polymer. A second solvent component of the solvent blend is preferably a good solvent for the active agent. The active agent is dissolved or dispersed in the solvent. Polymer matrix material is added to the agent-containing medium in an amount relative to the active agent that provides a product having the desired loading of active agent. Optionally, all of the ingredients of the microparticle product can be blended in the solvent blend medium together.

The preferred solvent system is a blend of at least two solvents. The solvents in the solvent blend are preferably:
(1) mutually miscible with one another,
(2) capable, when blended, of dissolving or dispersing the active agent,
(3) capable, when blended, of dissolving polymeric matrix material,
(4) chemically inert to the active agent,
(5) biocompatible,
(6) substantially immiscible with any quench liquid employed, e.g. having a solubility from about 0.1 to 25%, and
(7) solvents other than halogenated hydrocarbons.

An ideal solvent blend for encapsulation of an active agent should have a high solubility for the polymeric encapsulating agent of generally at least about 5 weight percent and, preferably, at least about 20 weight percent at 20°C. The upper limit of solubility is not critical, but if over about 50 weight percent of the solution is encapsulating polymer, the solution may become too viscous to handle effectively and conveniently. This is, of course, dependent on the nature of the encapsulating polymer and its molecular weight.

The solvent system, although substantially immiscible with the continuous phase process medium and any quenching liquid, which usually are water or water-based, preferably has a limited solubility therein. If the solvent system were infinitely soluble in the process medium, microparticles would be unable to form during the emulsion phase; if the solubility of the solvent system in an extractive quenching medium were too low, however, large quantities of quenching medium would be needed. Generally, solvent solubilities of from about 0.1 to about 25% in the process medium and any quench medium are acceptable for use herein. It will often be advantageous for the quench medium, if employed, to contain from about 70 to about 20 weight percent of the saturation point of the first solvent, i.e. the solvent of higher solubility in the quench medium, to control the rate of loss of the first solvent from the microparticles into the quench medium.

Added considerations in choosing a component of the solvent blend of the present invention include boiling point (i.e. the ease with which the solvents can be evaporated, if desired, to form finished product) and specific gravity (tendency of the discontinuous or oil phase to float during emulsifying and quenching). Finally, the solvent system should have low toxicity.

Generally, the solvent blend composition of two components will contain from about 25 to about 75 weight percent of the first solvent, and, correspondingly, from about 75 to about 25 weight percent of the second solvent.

Experiments using benzyl alcohol alone as the solvent did result in control of microparticle size as determined by inspection of the quench tank contents by optical microscopy. Upon drying, however, generally poor quality was found to have resulted. Often, recovery was difficult because of stickiness. Also, solvent residuals tended to be elevated. Using a solvent system of ethyl acetate and benzyl alcohol for the discontinuous or oil phase improved the microparticle quality and release characteristics.

The solvent blend of the present invention is preferably a blend of at least two of the following: an ester, an alcohol, and a ketone. Preferred esters are of the structure R¹COOR² where R¹ and R² are independently selected from the group consisting of alkyl moieties of from 1 to 4 carbon atoms, i.e. methyl, ethyl, propyl, butyl, and isomers thereof. The most preferred ester for use as one component of the solvent blend employed in the practice of the present invention is ethyl acetate.

Preferred alcohols are of the structure R³CH₂OH where R³ is selected from the group consisting of hydrogen, alkyl of from 1 to 3 carbon atoms, and aryl of from 6 to 10 carbon atoms. It is more preferred that R³ be aryl. The most preferred alcohol for use as one component of the solvent blend employed in the practice of the present invention is benzyl alcohol.

Preferred ketones are of the structure R⁴COR⁵ where R⁴ is selected from the group consisting of alkyl moieties of from 1 to 4 carbon atoms, i.e. methyl, ethyl, propyl, butyl, and isomers thereof and R⁵ is selected from the group consisting of alkyl moieties of from 2 to 4 carbon atoms, i.e. ethyl, propyl, butyl, and isomers thereof. The most preferred ketone for use as one component of the solvent blend employed in the practice of the present invention is methyl ethyl ketone.

The polymer matrix material of the microparticles of the present invention is biocompatible and biodegradable. The matrix material should be biodegradable in the sense that it should degrade by bodily processes to products readily disposable by the body and should not accumulate in the body. The products of the biodegradation should also be biocompatible with the body, as should any residual solvent that may remain in the microparticles.

Preferred examples of polymer matrix materials include poly(glycolic acid), poly(d,l-lactic acid), poly(l-lactic acid), copolymers of the foregoing, and the like. Various commercially available poly (lactide-co-glycolide) materials (PLGA) may be used in the method of the present invention. For example, poly (d,l-lactic-co-glycolic acid) is commercially available from Medisorb Technologies International L.P. e.g. a 50:50 poly (d,1 lactic co-glycolic acid) known as MEDISORB® 50:50 DL. This product has a mole percent composition of 50% lactide and 50% glycolide. Other suitable commercially available products are MEDISORB® 65:35 DL, 75:25 DL, 85:15 DL and poly(d,l-lactic acid) (d,l-PLA). Poly(lactide-co-glycolides) are also commercially available from Boehringer Ingelheim, e.g. PLGA 50:50 (Resomer® RG 502), PLGA 75:25 (Resomer® RG 752) and d,l-PLA (Resomer® RG 206), and from Birmingham Polymers. These copolymers are available in a wide range of molecular weights and ratios of lactic acid to glycolic acid.

The most preferred polymer for use in the practice of this invention is the copolymer, poly(d,l-lactide-coglycolide). It is preferred that the molar ratio of lactide to glycolide in such a copolymer be in the range of from about 85:15 to about 35:65, more especially about 75:25 to about 50:50, e.g. 85:15, 75:25, 65:35 or 50:50.

It will be understood that the problem addressed by the present invention is the undesirably short shelf-life engendered by the action of an active agent on the matrix polymer where the solvent, or at least one of the solvents of the solvent blend, used in making the microparticles remains in sufficient concentration in the finished product to exacerbate degrading interaction between the active agent and the polymer. This problem can be seen, for example, with an active agent having a basic moiety, such as risperidone, and a matrix polymer that has a group or linkage susceptible to base-catalysed hydrolysis.

The molecular weight of the polymeric matrix material is of some importance. The molecular weight should be high enough to permit the formation of satisfactory polymer coatings, i.e. the polymer should be a good film former. Usually, a satisfactory molecular weight is in the range of 5,000 to 500,000 daltons, preferably 50,000 to 400,000, more preferably 100,000 to 300,000, especially 100,000 to 200,000 and particularly about 150,000 daltons. However, since the properties of the film are also partially dependent on the particular polymeric matrix material being used, it is very difficult to specify a molecular weight range appropriate for all polymers. The molecular weight of a polymer is also important from the point of view of its influence upon the biodegradation rate of the polymer.

For a diffusional mechanism of drug release, the polymer should remain intact until all of the drug is released from the microparticles and then degrade. The drug can also be released from the microparticles as the polymeric excipient bioerodes. By an appropriate selection of polymeric materials a microparticle formulation can be made in which the resulting microparticles exhibit both diffusional release and biodegradation release properties. This is useful in affording multiphasic release patterns.

Those skilled in the art will comprehend that removal of residual solvent by the wash step of the present invention may have an effect upon the drug release rate, which may be either detrimental or beneficial, depending upon the circumstances. For example, where the residual solvent is acting as a plasticizer for the matrix polymer, the glass transition temperature may be seen to decrease, thereby possibly accelerating the release rate of the active agent. If, in a given situation, a faster release rate is desirable, this result will be beneficial. If, however, the rate becomes fast enough to negatively affect the desired action of the active agent with regard to the patient, it will be incumbent upon the formulator to employ means for alleviating the accelerated release rate. Such modifications of the process, when required, are within the capability of those of ordinary skill in the relevant arts and can be realized without undue experimentation.

The formulation of the present invention contains an active agent dispersed in the microparticle polymetric matrix material. The amount of such agent incorporated in the microparticles usually ranges from about 1 wt. % to about 90 wt. %, preferably 30 to 50 wt. %, more preferably 35 to 40 wt.%. By weight % is meant weight of agent as a percentage of the total weight of the microparticles. For example 10 wt. % agent may mean 10 parts agent and 90 parts polymer, by weight.

In carrying out the process of the present invention where this includes microparticle formation, the encapsulating polymer should be essentially 100% dissolved in the solvent or solvent blend at the time the solution is emulsified. The active agent can be dispersed or dissolved in the solvent or solvent blend at the time it is added to the continuous phase process medium. The content of normally solid material (active agent plus encapsulating polymer) in the solvent blend at the time it is first emulsified should be at least 5 weight percent and preferably at least 20 weight percent. Minimizing solvent in the discontinuous or oil phase provides a better quality microparticle and requires less extraction medium.

The microparticles can be mixed by size or by type so as to provide for the delivery of active agent to the patient in a multiphasic manner and/or in a manner that provides different active agents to the patient at different times, or a mixture of active agents at the same time. For example, secondary antibiotics, vaccines, or any desired active agent, either in microparticle form or in conventional, unencapsulated form can be blended with a primary active agent and provided to the patient.

The mixture of ingredients in the discontinuous or oil phase solvent system is emulsified in a continuous-phase processing medium; the continuous phase medium being such that a dispersion of microparticles containing the indicated ingredients is formed in the continuous-phase medium.

Although not absolutely necessary, it is preferred to saturate the continuous phase process medium with at least one of the solvents forming the discontinuous or oil phase solvent system. This provides a stable emulsion, preventing transport of solvent out of the microparticles prior to quenching. Similarly, a vacuum may be applied as in U.S. Patent No. 4,389,330. Where ethyl acetate and benzyl alcohol are the components of the solvent system, the aqueous or continuous phase of the emulsion preferably contains 1 to 8 weight percent ethyl acetate and 1 to 4 weight percent benzyl alcohol.

Usually, a surfactant or a hydrophilic colloid is added to the continuous phase processing medium to prevent the solvent microdroplets from agglomerating and to control the size of the solvent microdroplets in the emulsion. Examples of compounds that can be used as surfactants or hydrophilic colloids include, but are not limited to, poly(vinyl alcohol), carboxymethyl cellulose, gelatin, poly(vinyl pyrrolidone), Tween^{®} 80, Tween^{®} 20, and the like. The concentration of surfactant or hydrophilic colloid in the process medium should be sufficient to stabilize the emulsion and will affect the final size of the microparticles. Generally the concentration of the surfactant or hydrophilic colloid in the process medium will be from about 0.1% to about 10% by weight based on the process medium, depending upon the surfactant or hydrophilic colloid, the discontinuous or oil phase solvent system, and the processing medium used. A preferred dispersing medium combination is a 0.1 to 10 wt.%, more preferably 0.5 to 2 wt.%, solution of poly(vinyl alcohol) in water.

The emulsion can be formed by mechanical agitation of the mixed phases or by adding small drops of the discontinuous phase that contains active agent and wall forming material to the continuous phase processing medium. The temperature during the formation of the emulsion is not especially critical, but can influence the size and quality of the microparticles and the solubility of the active agent in the continuous phase. Of course, it is desirable to have as little of the active agent in the continuous phase as possible. Moreover, depending upon the solvent blend and continuous-phase processing medium employed, the temperature must not be too low or the solvent and processing medium may be solid or become too viscous for practical purposes. On the other hand, it must not be so high that the processing medium will evaporate or that the liquid processing medium will not be maintained. Moreover, the temperature of the emulsion cannot be so high that the stability of the particular active agent being incorporated in the microparticles is adversely affected. Accordingly, the dispersion process can be conducted at any temperature that maintains stable operating conditions, preferably from about 20°C to about 60°C, depending upon the active agent and excipient selected.

As stated above, in order to create microparticles containing an active agent, an organic or oil (discontinuous) phase and an aqueous phase are combined. The organic and aqueous phases are largely or substantially immiscible, with the aqueous phase constituting the continuous phase of the emulsion. The organic phase includes the active agent as well as the wall forming polymer, i.e. the polymeric matrix material. The organic phase is prepared by dissolving or dispersing the active agent(s) in the organic solvent system of the present invention. The organic phase and the aqueous phase are preferably combined under the influence of mixing means, preferably a static mixer. Preferably, the combined organic and aqueous phases are pumped through a static mixer to form an emulsion comprising microparticles containing active agent encapsulated in polymeric matrix material, and then into a large volume of quench liquid to obtain microparticles containing the active agent encapsulated in the polymeric matrix material. Preferably, the microparticles are then stirred in a tank containing a quench solution in order to remove most of the organic solvent from the microparticles, resulting in the formation of hardened microparticles. An especially preferred method of mixing with a static mixer is disclosed by Ramstack *et al.* in WO95/13799.

One advantage of using a static mixer is that accurate and reliable scaling from laboratory to commercial batch sizes can be done while achieving a narrow and well defined size distribution of microparticles containing biologically or pharmaceutically active agents. A further advantage of this method is that the same equipment can be used to form microparticles containing active agents of a well defined size distribution for varying batch sizes. In addition to improving process technology, static mixers are low maintenance and less spacious than dynamic mixers, and they have low energy demands and comparatively low investment costs.

Following the movement of the microparticles from the static mixer and entrance into the quench tank, the continuous-phase processing medium is diluted and much of the solvent in the microparticles is removed by extraction. In this extractive quench step, the microparticles can be suspended in the same continuous-phase processing medium used during emulsification, with or without hydrophilic colloid or surfactant, or in another liquid. The extraction medium removes a significant portion of the solvent from the microparticles, but does not dissolve them. During the extraction, the extraction medium containing dissolved solvent can, optionally, be removed and replaced with fresh extraction medium.

After the quench step has been completed, the microparticles can be isolated as stated above, and then may, if desired, be dried by exposure to air or by other conventional drying techniques, such as, vacuum drying, drying over a desiccant, or the like. This process is very efficient in encapsulating an active agent since core loadings of up to about 80 wt. %, preferably up to about 50 wt. can be obtained.

When a solvent blend is used to form the organic or oil phase droplets in the emulsion, one of the solvents in the solvent blend will be extracted in the quench step more quickly than the other solvent, e.g. the first solvent, ethyl acetate, in the case of the preferred ethyl acetate/benzyl alcohol blend. Thus, high residuals of the second solvent (here, benzyl alcohol) are left behind. Owing to the high boiling point of benzyl alcohol, it is not easily removed by exposure of the microparticles to air or other conventional evaporative means. To improve the efficiency of this procedure, some of the more rapidly extracted solvent can be added to the quench extraction medium prior to addition of the emulsion. The concentration of the more-rapidly-extracted solvent in the quench extraction medium generally is from about 20 to about 70% of the saturation point of the solvent in the medium at the temperature to be used for the extraction. Thus when the emulsion is added to the quench liquid, extraction of the more rapidly extracted solvent is retarded and more of the second, more slowly extracted, solvent is removed.

The exact amount of this more-rapidly-extracted solvent "spike" added to the quench liquid is of importance to final microparticle quality. Too much solvent (i.e. near the saturation point) results in porous microparticles with active agent visible on the surface, causing what may be an undesirably high rate of release. Too little solvent in the quench medium results in high residual level of the more-slowly-extracted solvent and poor microparticle quality. The temperature of the quench medium is also important as it affects solvent solubility and rate of extraction.

Both temperature and amount of solvent spike can be adjusted to contribute beneficially to the final desired product characteristics, i.e. highly porous, quick releasing microparticles, or slow releasing microparticles having a low porosity.

The quench liquid can be plain water, a water solution, or other suitable liquid, the volume, amount, and type of which depends on the solvents used in the emulsion phase. The quench liquid is preferably water. Generally, the quench liquid volume is on the order of 10 times the saturated volume (i.e. 10 times the quench volume needed to absorb completely the volume of solvent in the emulsion). Depending on the solvent system, however, quench volume can vary from about 2 to about 20 times the saturated volume. Additionally, it is convenient to describe the quench volume requirement relative to batch size (microparticle product). This ratio is an indication of efficiency of the extraction step and, in some cases, dictates the batch size for a given set of equipment. The larger the ratio, the more volume is required per product weight. On the other hand, with a smaller ratio, more product can be obtained from the same amount of quench volume. This ratio can vary from about 0.1 to about 10 liters of quench volume per gram of microparticles produced. Processes with a ratio of less than about 1 liter per gram are preferred.

When using the preferred solvent combination of benzyl alcohol and ethyl acetate, the ethyl acetate content of the quench liquid appears to affect the residual solvent level in the product microparticles. At low ethyl acetate contents in the quench liquid, the benzyl alcohol residuals in the microparticles are high while ethyl acetate may be almost non-detectable. At high ethyl acetate contents in the quench liquid, more ethyl acetate may be retained by the microparticles than benzyl alcohol. At a quench volume of about 1 liter per gram of active agent and polymeric encapsulating material being quenched, about 2-4 weight percent ethyl acetate in the quench liquid is optimal at 0-10°C.

After the quenching step, the microparticles are isolated from the aqueous quench solution by any convenient means of separation - the fluid can be decanted from the microparticles or the microparticle suspension can be filtered, for example, a sieve column can be used. Various other combinations of separation techniques can be used, if desired. Filtration is preferred.

The filtered microparticles are then subjected to the washing step of the present invention in order to reduce still further the level of residual solvent(s) therein, preferably to a level in the range of from about 0.2 to 2.0%. In practice, it has been found that, in the preferred ethyl acetate/benzyl alcohol dual solvent case, residual benzyl alcohol levels are still generally in the 4-8% range without the washing step of the present invention. This level of residual solvent in the microparticles appears to be sufficient to accelerate the degradation process, thereby reducing shelf-life. Degradation of the microparticles can occur, for example, by undesired hydrolysis of the hydrolyzable linkages of a matrix polymer by a basic active agent. Thus, the washing step(s) of the present invention are employed to reduce the residual benzyl alcohol or other solvent content in the microparticles to retard the degradation process.

As stated above, the wash solution comprises either water alone or, preferably, water and a solvent miscible therewith that is also a good solvent for the residual solvent in the microparticles. Where, as in the preferred process of the present invention, the residual solvent is benzyl alcohol, C₁-C₄aliphatic alcohols are preferred for use in the wash solution. These alcohols are methanol, ethanol, propanol, butanol, and isomers of the foregoing. The most preferred alcohol is ethanol.

The concentration of the alcohol in the wash solution can vary depending upon the particular circumstances. Generally, the alcohol will comprise less than 50% by weight with a lower limit of about 5%. Thus, a preferred range for the alcohol concentration with normally be from about 5% to about 50% by weight. More preferably, the concentration will lie in the range of from about 15% to about 30%.

The temperature of the wash solution is also important to the efficiency of the washing step. Generally, increasing the temperature will decrease the time needed for the wash to lower the remaining residual content to the desired level.

On the other hand, too high a temperature can be detrimental in that the softening temperature of the matrix polymer of the microparticles may be approached or exceeded, thereby causing clumping or stickiness. Conversely, too low a temperature may cause the matrix material to become too hard, thereby retarding the rate at which the residuals can be extracted, whereby the process may become prohibitively expensive. It has been found that a temperature range of from about 5°C to about 40°C is convenient and effective. Preferably, the temperature employed will bracket room temperature, i.e. from about 10°C to about 30°C. Where water alone is used as the wash solvent, it will be employed at an elevated temperature, i.e. above room temperature, preferably in a range of from about 25°C to about 40°C, most preferably, about 37°C.

Normally, it will be desirable to employ more than one wash step, typically two or three. After each such step, the microparticles will be separated from the wash solution by well-known separation means, e.g. filtration, decantation, centrifugation, and the like. Filtration is preferred.

After each separation step, the microparticles can, if desired, be fully or partially dried employing conventional drying means at temperatures substantially similar to those of the previous wash solution. The use of dry compressed air at temperatures ranging from about 10°C to about 30°C has been found especially useful and convenient and is preferred.

The microparticle product is usually made up of particles of a spherical shape, although sometimes the microparticles may be irregularly shaped. The microparticles can vary in size, ranging from submicron to millimeter diameters. Preferably, microparticles of 1-500 microns, more preferably, 25-180 microns, are prepared, whereby administration of the microparticles to a patient can be carried out with a standard gauge needle.

Preferably, the drug-loaded microparticles are dispensed to patients in a single administration, releasing the drug in a constant or pulsed manner into the patient and eliminating the need for repetitive injections.

The active agent bearing microparticles are obtained and stored as a dry material. Prior to administration to a patient, the dry microparticles can be suspended in an acceptable pharmaceutical liquid vehicle, such as a 2.5 wt. % solution of carboxymethyl cellulose, whereupon the suspension is injected into the body.

The microparticles can be mixed by size or by type so as to provide for the delivery of active agent to the patient in a multiphasic manner and/or in a manner that provides different active agents to the patient at different times, or a mixture of active agents at the same time. For example, secondary antibiotics, vaccines, or any desired active agent, either in microparticle form or in conventional, unencapsulated form can be blended with a primary active agent and provided to the patient.

For those materials that have no groups detrimental to the integrity of the matrix polymer, the additional washing step(s) of the present invention may prove beneficial in ways, such as, controlling the release characteristics of active agent *in vivo* or reducing an undesirable or possibly harmful solvent.

The invention will now be illustrated further by the following non-limiting Examples and with reference to the accompanying drawings, in which:-
Figure 1 is a graph showing the reduction in benzyl alcohol levels in a finished product as a function of ethanol concentration (5%; 15%; 20%; 25%) in an ethanol:water wash;
Figure 2 is a graph showing the impact of microparticle concentration on the level of residual benzyl alcohol (BA) in the finished product;
Figure 3 is a graph showing the impact of temperature of the wash step on the level of residual benzyl alcohol (BA) in the finished product; and
Figure 4 is a graph showing the effect of the level of residual solvent (benzyl alcohol) on the decay in molecular weight of the polymeric matrix.

### Example 1

In a typical 125 gram batch, 75 g of 75:25 Medisorb^{®} lactide:glycolide copolymer and 50 g of risperidone are dissolved in 275 g of benzyl alcohol and 900.25 g of ethyl acetate as the organic phase. The aqueous phase comprises 90.0 g of polyvinyl alcohol, 8910 g of water, 646.4 g of ethyl acetate, and 298.3 g of benzyl alcohol. The organic and aqueous phases are pumped through a static mixer to form an emulsion. The resulting emulsion is passed into a quench liquid comprising 17 kg of water, 4487.8 g of ethyl acetate, 371.0 g of sodium carbonate, and 294.0 g of sodium bicarbonate. After 20 hours at approximately 10°C, the resulting microspheres are then filtered and washed with a first wash of 11.25 kg of ethanol and 33.75 kg of water for 2 hours at 10°C. The microspheres are then filtered and washed with a solution of 11.25 kg of ethanol and 33.75 kg of water for 6 hours at 25°C. A third wash of 756 g of citric acid, 482 g of sodium phosphate, and 45.0 kg of water is then applied at 25°C for one hour to the filtered product. The product is then rinsed with water, filtered, and dried. Three batches produced according to this procedure provide risperidone contents of 37.4%, 37.0%, and 36.6% by weight. Benzyl alcohol levels were 1.36%, 1.26%, and 1.38% by weight. Ethyl acetate levels were 0.09%, 0.08%, and 0.09% by weight.

### Example 2

### Effect of the Wash Process on Microparticle Characteristics

A sample of risperidone-loaded microspheres was subjected to a series of wash experiments to determine the impact on finished product characteristics and identify favourable wash conditions. The sample comprised risperidone encapsulated in a 75:25 Medisorb^{®} lactide:glycolide copolymer. The drug content was 36.8% by weight, and the benzyl alcohol level was about 5.2% by weight prior to the washing experiments. The microspheres ware transferred into the wash media, samples were withdrawn at selected time periods and vacuum dried.

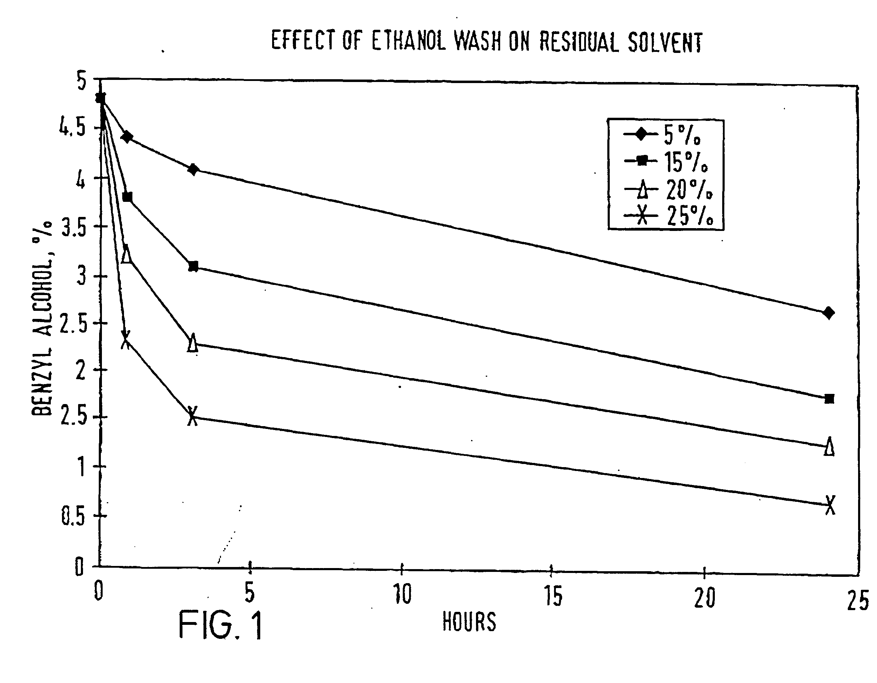

Figure 1 shows the reduction in benzyl alcohol levels in the finished product as a function of ethanol concentrations (5%; 15%; 20%; and 25%) in the ethanol:water wash. Higher ethanol levels afforded lower residual benzyl alcohol in the finished product.

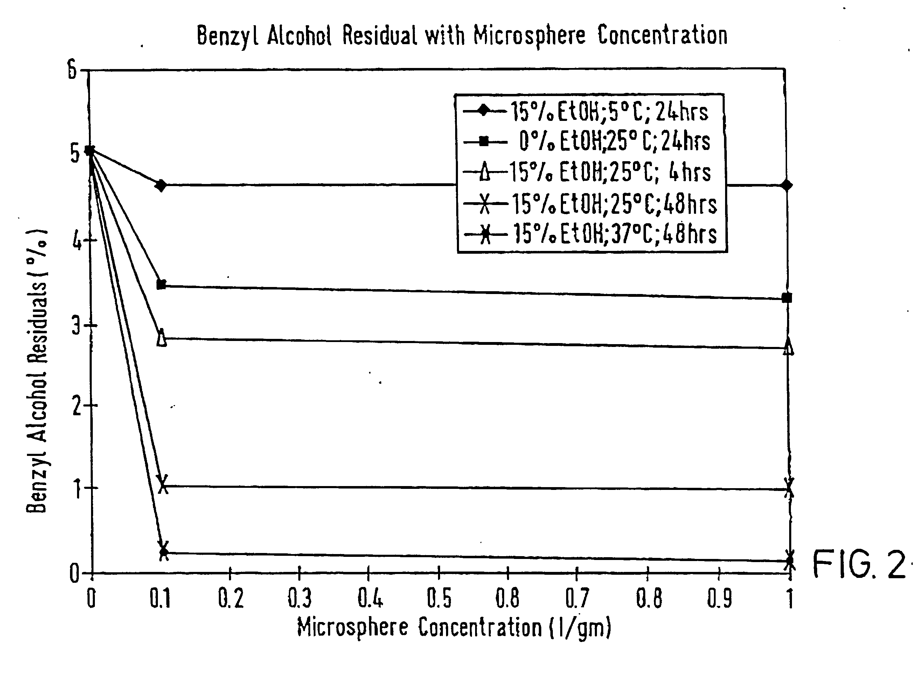

Figure 2 shows that in the range of 0.1 to 1.0 liters of solution per gram of microspheres, the concentration of microspheres in the wash step does not influence the level of residual benzyl alcohol (BA) in the finished product.

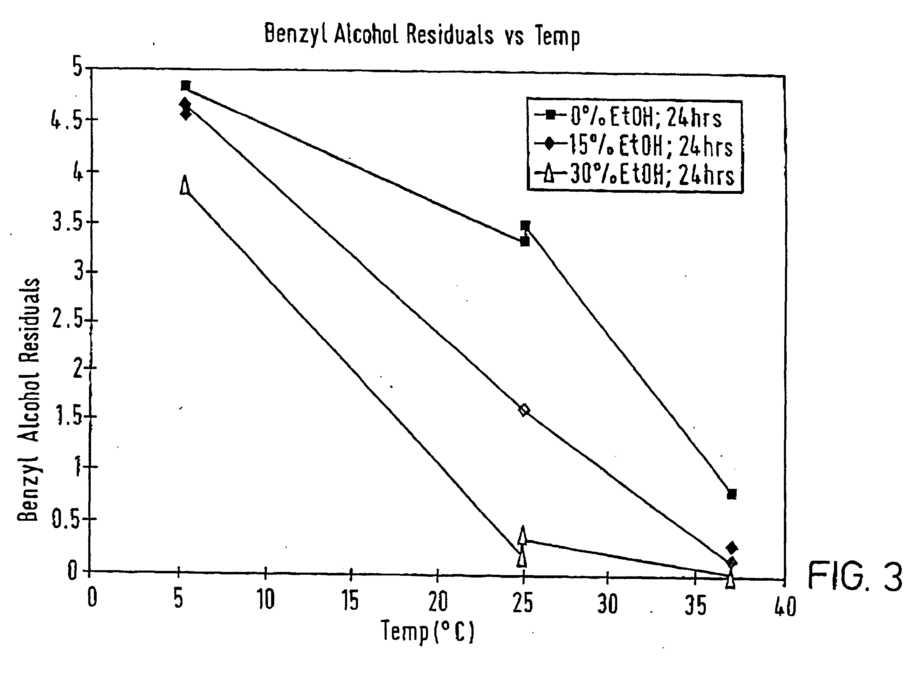

Figure 3 shows the impact of temperature of the wash step on the level of residual benzyl alcohol in the finished product.

Table 1 shows an increase in glass-transition temperature (Tg) of the finished microspheres as the wash time increases, and as the concentration of ethanol increases and the corresponding concentration of benzyl alcohol decreases.

| **Table 1 Effect of Ethanol Wash Time and Concentration on Glass Transition Temperature, T_{g}** | | | | |
|---|---|---|---|---|
| Wash Time (Hours) | 5% Ethanol | 15% Ethanol | 20% Ethanol | 25% Ethanol |
| 0.75 | 24.2°C | 26.5°C | 30.1°C | 30.8°C |
| 3 | 26.5°C | 26.5°C | 32.5°C | 35.1°C |
| 24 | 30.9°C | 28.7°C | 37.3°C | 40.1°C |

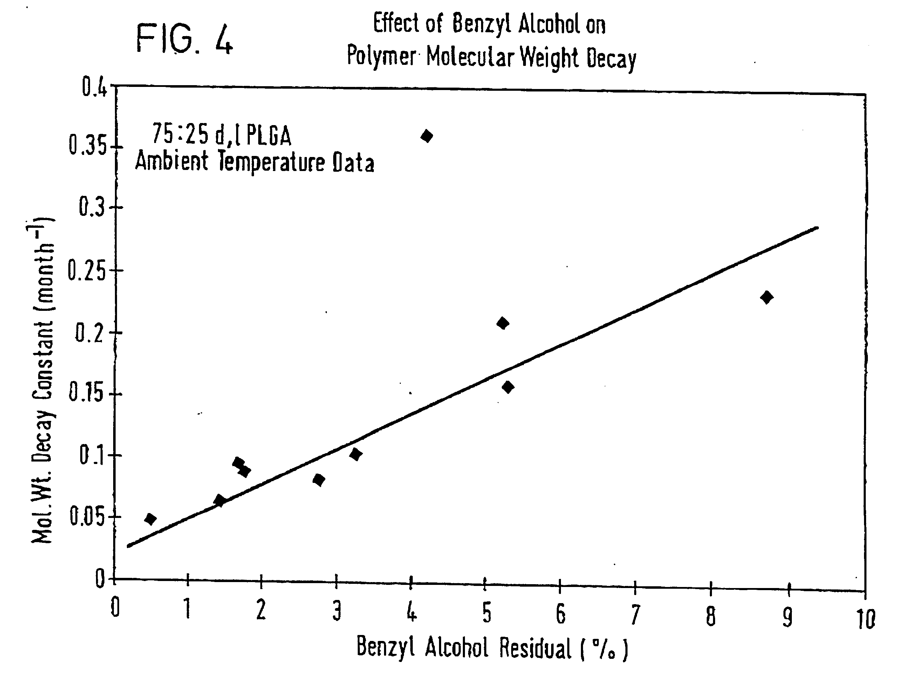

Risperidone-loaded microspheres with various levels of benzyl alcohol were placed in stability studies at room temperature. Figure 4 demonstrates that the degradation process as measured by the rate of hydrolysis of the biodegradable, biocompatible polymer is strongly influenced by the level of residual solvent in the finished product. The molecular weight decay constant was plotted versus residual benzyl alcohol level for ten different microsphere samples.

## Claims

1. Microparticles of a biodegradable biocompatible polymeric matrix containing an active agent and an organic solvent free from halogenated hydrocarbons, said residual organic solvent being present in said microparticles at 2% or less of the total weight of said microparticles wherein the active agent is selected from the group of risperidone, 9-hydroxy-risperidone, and pharmaceutically acceptable salts thereof.

2. Microparticles according to claim 1 wherein the active agent is risperidone.

3. Microparticles according to claim 1 wherein the active agent is 9-hydroxy-risperidone.

4. Microparticles as claimed in any one of claims 1 to 3 prepared by a process comprising either:
(a) contacting said microparticles with water and maintaining said water at a temperature in the range of from 25°C to 40°C during at least part of the time that it is in contact with said microparticles,
or:
(b) contacting said microparticles with an aqueous solvent system comprising water and a water-miscible solvent for said organic solvent.

5. Microparticles as claimed in any one of claims 1 to 4 wherein the microparticles have a diameter ranging between 25 to 180 microns.

6. Microparticles as claimed in any one of claims 1 to 5, wherein said polymeric matrix is a copolymer of poly(glycolic acid) and poly(d, l-lactic acid).

7. Microparticles as claimed in any one of claims 1 to 6 wherein said residual organic solvent comprises benzyl alcohol present in the range of from 0.5 to 1.5% by weight.

8. Microparticles as claimed in any one of claims 1 to 7 wherein said organic solvent is a blend of at least two solvents.

9. Microparticles as claimed in any one of claims 1 to 8 wherein said organic solvent is a blend of ethyl acetate and benzyl alcohol.

10. Microparticles as claimed in any one of claims 1 to 9 having a diameter ranging in size from 25 to 180 microns comprising: a copolymer of poly(glycolic acid) and poly(d,l-lactic acid) wherein the molar ratio of lactide to glycolide is in the range of from 85:15 to 50:50 and having dispersed or dissolved therein from 35 to 40wt% of an active agent selected from risperidone, 9-hydroxy-risperidone and pharmaceutically acceptable salts thereof, and from 0.5 to 1.5% by weight of benzyl alcohol.

11. A pharmaceutical composition comprising microparticles according to any one of claims 1 to 10 together with at least one pharmaceutically acceptable carrier or excipient.

12. The use of microparticles as claimed in claim 1 to 10 for the manufacture of a medicament for use in a method of diagnosis or therapy.

13. The use of a pharmaceutical composition as claimed in claim 11 for the manufacture of a medicament for use in a method of diagnosis or therapy.

## Patentansprüche

1. Mikroteilchen aus einer biologisch abbaubaren biokompatiblen Polymermatrix mit einem Wirkstoffe und einem von halogenierten Kohlenwasserstoffen freien organischen Lösungsmittel, wobei das restliche organische Lösungsmittel in den Mikroteilchen in einer Menge von 2% oder weniger, bezogen auf das Gesamtgewicht der Mikroteilchen, beträgt, wobei der Wirkstoff , aus der Gruppe bestehend aus Risperidon, 9-Hydroxyrisperidon und pharmazeutisch unbedenklichen Salzen davon ausgewählt ist.

2. Mikroteilchen nach Anspruch 1, wobei es sich bei dem Wirkstoff um Risperidon handelt.

3. Mikroteilchen nach Anspruch 1, wobei es sich bei dem Wirkstoff um 9-Hydroxyrisperidon handelt.

4. Mikroteilchen nach einem der Ansprüche 1 bis 3, hergestellt nach einem Verfahren, bei dem man entweder:
(a) die Mikroteilchen mit Wasser in Berührung bringt und das Wasser während mindestens eines Teils der Zeit, während der es mit den Mikroteilchen in Berührung kommt, bei einer Temperatur im Bereich von 25°C bis 40°C hält
oder:
(b) die Mikroteilchen mit einem wäßrigen Lösungsmittelsystem, das Wasser und ein wassermischbares Lösungsmittel für das organische Lösungsmittel enthält, in Berührung bringt.

5. Mikroteilchen nach einem der Ansprüche 1 bis 4 mit einem Durchmesser im Bereich von 25 bis 180 Mikron.

6. Mikroteilchen nach einem der Ansprüche 1 bis 5, wobei es sich bei der Polymermatrix um ein Copolymer von Poly(glykolsäure) und Poly(d,1-milchsäure) handelt.

7. Mikroteilchen nach einem der Ansprüche 1 bis 6, wobei das restliche organische Lösungsmittel Benzylalkohol, der in einer Menge im Bereich von 0,5 bis 1,5 Gew.-% vorliegt, umfaßt.

8. Mikroteilchen nach einem der Ansprüche 1 bis 7, wobei es sich bei dem organischen Lösungsmittel um eine Mischung aus mindestens zwei Lösungsmitteln handelt.

9. Mikroteilchen nach einem der Ansprüche 1 bis 8, wobei es sich bei dem organischen Lösungsmittel um eine Mischung aus Essigsäureethylester und Benzylalkohol handelt.

10. Mikroteilchen nach einem der Ansprüche 1 bis 9 mit einem Durchmesser im Bereich von 25 bis 180 Mikron, enthaltend: ein Copolymer von Poly-(glykolsäure) und Poly(d,1-milchsäure) mit einem Lactid/Glykolid-Molverhältnis im Bereich von 85:15 bis 50:50, worin 35 bis 40 Gew.-% eines unter Risperidon, 9-Hydrokyrisperidon und pharmazeutisch unbedenklichen Salzen davon ausgewählten Wirkstoffs dispergiert oder gelöst sind, und 0,5 bis 1,5 Gew.-% Benzylalkohol.

11. Pharmazeutische Zusammensetzung, enthaltend Mikroteilchen nach einem der Ansprüche 1 bis 10 zusammen mit mindestens einem pharmazeutisch unbedenklichen Träger oder Hilfsstoff.

12. Verwendung von Mikroteilchen nach Anspruch 1 bis 10 zur Herstellung eines Arzneimittels zur Verwendung bei einem Diagnose- oder Therapieverfahren.

13. Verwendung einer pharmazeutischen Zusammensetzung nach Anspruch 11 zur Herstellung eines Arzneimittels zur Verwendung bei einem Diagnose- oder Therapieverfahren.

## Revendications

1. Microparticules d'une matrice polymère biocompatible biodégradable contenant un agent actif et un solvant organique exempt d'hydrocarbures halogénés, ledit solvant organique résiduel étant présent dans lesdites micro-particules à un taux de 2 % ou moins du poids total desdites microparticules, où l'agent actif est choisi parmi le groupe constitué de la rispéridone, de la 9-hydroxyrispéridone, et de leurs sels pharmaceutiquement acceptables.

2. Microparticules selon la revendication 1, dans lesquelles l'agent actif est la rispéridone.

3. Microparticules selon la revendication 1, dans lesquelles l'agent actif est la 9-hydroxy-rispéridone.

4. Microparticules selon l'une quelconque des revendications 1 à 3, préparées par un procédé comprenant soit:
(a) la mise en contact desdites microparticules avec de l'eau et le maintien de ladite eau à une température dans la plage de 25°C à 40°C, durant au moins une partie de la période pendant laquelle elle est en contact avec lesdites microparticules,
soit :
(b) la mise en contact desdites microparticules avec un système de solvant aqueux comprenant de l'eau et un solvant miscible à l'eau pour ledit solvant organique.

5. Microparticules selon l'une quelconque des revendications 1 à 4, où les microparticules présentent un diamètre s'échelonnant entre 25 et 180 microns.

6. Microparticules selon l'une quelconque des revendications 1 à 5, dans lesquelles ladite matrice polymère est un copolymère de l'acide polyglycolique et de l'acide poly(d,1-lactique).

7. Microparticules selon l'une quelconque des revendications 1 à 6, dans lesquelles ledit solvant organique résiduel comprend de l'alcool benzylique présent dans la plage de 0,5 à 1,5 % en poids.

8. Microparticules selon l'une quelconque des revendications 1 à 7, dans lesquelles ledit solvant organique est un mélange d'au moins deux solvants.

9. Microparticules selon l'une quelconque des revendications 1 à 8, dans lesquelles ledit solvant organique est un mélange d'acétate d'éthyle et d'alcool benzylique.

10. Microparticules selon l'une quelconque des revendications 1 à 9, présentant un diamètre. s'échelonnant dans la plage de 25 à 180 microns, comprenant : un copolymère de l'acide polyglycolique et de l'acide poly(d,1-lactique), où le rapport molaire entre le lactide et le glycolide est dans la plage de 85:15 à 50:50, et comprenant, à l'état dispersé ou dissous, de 35 à 40 % en poids d'un agent actif choisi parmi la rispéridone, la 9-hydroxyrispéridone et leurs sels pharmaceutiquement acceptables, et de 0,5 à 1,5 % en poids d'alcool bénzylique.

11. Composition pharmaceutique comprenant des microparticules selon l'une quelconque des revendications, 1 à 10, conjointement avec au moins un support ou un excipient pharmaceutiquement acceptable.

12. Utilisation de microparticules selon les revendications 1 à 10, pour la fabrication d'un médicament destiné à être utilisé dans une méthode de diagnostic ou de thérapie.

13. Utilisation d'une composition pharmaceutique selon la revendication 11, pour la fabrication d'un médicament destiné à être utilisé dans une méthode de diagnostic ou de thérapie.
